# EUROPEAN PATENT APPLICATION

(11) **EP 1 671 576 A1**
(43) Date of publication of application: **21.06.2006**
(21) Application number: 05257913.3
(22) Date of filing: 16.12.2005
(51) Int. Cl.: A61B 1/00

(54) **Endoscopic device with retaining means for attaching an auxiliary device**

(30) Priority: 17.12.2004 US 15847
(71) Applicant: ETHICON ENDO-SURGERY, INC., Cincinnati, Ohio 45242 (US)
(72) Inventor: Hunt, John Vincent, Cincinnati Ohio 45241 (US)
(74) Representative: Tunstall, Christopher Stephen

(57) **Abstract**

An endoscope includes an endoscope handpiece having a working channel opening and having either a retainer, an endoscope-adjunct-device actuation carriage, or an endoscope-adjunct-device actuation cylinder associated with a handle or a cable of an endoscope adjunct device, wherein the cable is insertable into the working channel opening. Examples of endoscope adjunct devices include, without limitation, a grasper, biopsy forceps, and a snare. An endoscope component includes either an endoscope-handpiece retainer, an endoscope-adjunct-device actuation carriage, or an endoscope-adjunct-device actuation cylinder which is attachable to an endoscope handpiece.

## Description

**Field of the Invention**

The present invention is related generally to medical instruments, and more particularly to an endoscope and to a component attachable to an endoscope handpiece.

**Background of the Invention**

Endoscopes are known medical instruments having a handpiece, having a flexible tube which extends from the distal end of the handpiece and which is insertable into a body opening, and having monitor cabling which extends from the handpiece for connection to a monitor. The handpiece typically includes knobs and buttons to allow a first operator of the endoscope to manipulate the distal end portion of the flexible tube in a body passageway, to deliver air or water to the distal end portion of the flexible tube, to supply suction to the distal end portion of the flexible tube, to take photo snapshots from the distal end portion of the flexible tube, etc. The handpiece also includes a working channel opening which allows a second operator to hold the handle of an endoscope adjunct device (such as a grasper, biopsy forceps, or a snare) while inserting a cable of the endoscope adjunct device into the working channel opening and moving the cable to the distal end of the flexible tube whereupon the second operator activates the endoscope adjunct device to operate an endoscope-adjunct-device end effector located at the distal end portion of the cable.

Still, scientists and engineers continue to seek improved endoscopes.

**Summary**

A first expression of a first embodiment of the invention is for an endoscope including an endoscope handpiece. The endoscope handpiece includes a working channel opening and a retainer. The retainer is adapted to manually attach thereto, and manually remove therefrom, a handle of an endoscope adjunct device having a cable insertable into the working channel opening.

A second expression of a first embodiment of the invention is for an endoscope component including an endoscope-handpiece retainer attachable to an endoscope handpiece. The endoscope handpiece includes a working channel opening. The endoscope-handpiece retainer is adapted to manually attach thereto, and manually remove therefrom, a handle of an endoscope adjunct device having a cable insertable into the working channel opening.

A first expression of a second embodiment of the invention is for an endoscope including an endoscope handpiece. The endoscope handpiece includes a working channel opening and an endoscope-adjunct-device actuation carriage. The endoscope-adjunct-device actuation carriage is adapted to manually attach thereto, and manually remove therefrom, a handle of an endoscope adjunct device having a cable insertable into the working channel opening. The endoscope-adjunct-device actuation carriage is adapted to allow a user of the endoscope to remotely manipulate the handle of the endoscope adjunct device to activate the endoscope adjunct device when the handle is attached to the endoscope-adjunct-device actuation carriage.

A second expression of a second embodiment of the invention is for an endoscope component including an endoscope-adjunct-device actuation carriage attachable to an endoscope handpiece. The endoscope handpiece includes a working channel opening. The endoscope-adjunct-device actuation carriage is adapted to manually attach thereto, and manually remove therefrom, a handle of an endoscope adjunct device having a cable insertable into the working channel opening. The endoscope-adjunct-device actuation carriage is adapted to allow a user of the endoscope to remotely manipulate the handle of the endoscope adjunct device to activate the endoscope adjunct device when the handle is attached to the endoscope-adjunct-device actuation carriage.

A first expression of a third embodiment of the invention is for an endoscope including an endoscope handpiece. The endoscope handpiece includes a working channel opening and an endoscope-adjunct-device actuation cylinder. The endoscope-adjunct-device actuation cylinder is adapted to manually operatively attach thereto, and manually operatively remove therefrom, a proximal end of a cable of an endoscope adjunct device. The cable has a distal end insertable into the working channel opening. The endoscope-adjunct-device actuation cylinder is adapted for remote activation by a user of the endoscope.

A second expression of a third embodiment of the invention is for an endoscope component including an endoscope-adjunct-device actuation cylinder attachable to an endoscope handpiece. The endoscope handpiece includes a working channel opening. The endoscope-adjunct-device actuation carriage is adapted to manually operatively attach thereto, and manually operatively remove therefrom, a proximal end of a cable of an endoscope adjunct device. The cable has a distal end insertable into the working channel opening. The endoscope-adjunct-device actuation cylinder is adapted for remote activation by a user of the endoscope.

Several benefits and advantages are obtained from one or more of the expressions of embodiments of the invention. In one application of the first and/or second expression of the first embodiment, by having a retainer which holds the handle of an existing endoscope adjunct device, a single user of the endoscope can operate the endoscope and more conveniently operate the endoscope adjunct device. In one application of the first and/or second expression of the second embodiment, by having an endoscope-adjunct-device actuation carriage, a single user of the endoscope can operate the endoscope and remotely manipulate the handle of the endoscope adjunct device to activate the device. In one application of the first and/or second expression of the third embodiment, by having an endoscope-adjunct-device actuation cylinder, a single user of the endoscope can operate the endoscope and remotely manipulate the cable of a handle-less endoscope adjunct device to activate the device.

**Brief Description of the Figures**

FIGURE 1 is a schematic, perspective view of a first embodiment of the invention showing an endoscope and an endoscope adjunct device removed from a retainer of the endoscope handpiece;

FIGURE 2 is a schematic, perspective view of a second embodiment of the invention showing a portion of an endoscope and an endoscope adjunct device removed from an endoscope-adjunct-device actuation carriage of the endoscope handpiece; and

FIGURE 3 is a schematic, perspective view of a third embodiment of the invention showing a portion of an endoscope and a cable of an endoscope adjunct device operatively attached to an endoscope-adjunct-device actuation cylinder of the endoscope handpiece.

**Detailed Description**

Before explaining several embodiments of the present invention in detail, it should be noted that each embodiment is not limited in its application or use to the details of construction and arrangement of parts and steps illustrated in the accompanying drawings and description. The illustrative embodiments of the invention may be implemented or incorporated in other embodiments, variations and modifications, and may be practiced or carried out in various ways. Furthermore, unless otherwise indicated, the terms and expressions employed herein have been chosen for the purpose of describing the illustrative embodiments of the present invention for the convenience of the reader and are not for the purpose of limiting the invention.

It is further understood that any one or more of the following-described embodiments, expressions of embodiments, examples, etc. can be combined with any one or more of the other following-described embodiments, expressions of embodiments, examples, etc.

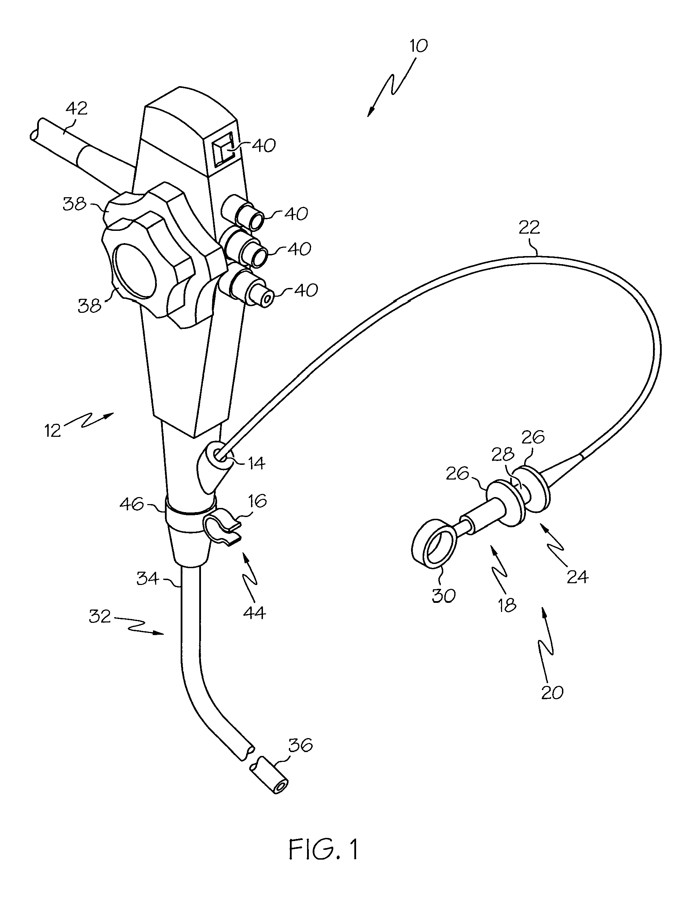

A first embodiment of the invention is shown in Figure 1. A first expression of the embodiment of Figure 1 is for an endoscope 10 including an endoscope handpiece 12 having a working channel opening 14 and a retainer 16, The retainer 16 is adapted to manually attach thereto, and manually remove therefrom, a handle 18 of an endoscope adjunct device 20 having a cable 22 insertable into the working channel opening 14. In one construction, the retainer 16 is a spring clip. Other constructions are left to the artisan.

In one application of the first expression of the embodiment of Figure l, a single user of the endoscope 10 can operate the endoscope 10 and can feed the cable 22 of the endoscope adjunct device 20 into the working channel opening 14 without the handle 18 of the endoscope adjunct device 20 dangling about and without requiring a separate user for the endoscope adjunct device 20. In one variation, at least one (and preferably some and more preferably all) existing endoscope adjunct device is employable with the retainer without having to redesign the handle of the endoscope adjunct device.

In one employment of the first expression of the embodiment of Figure 1, the endoscope adjunct device 20 is chosen from the group consisting of a grasper, biopsy forceps (shown), and a snare. An endoscope adjunct device is a medical instrument having a cable (i.e., at least one cable) insertable into the working channel opening 14 of the endoscope handpiece 12.

In one arrangement of the first expression of the embodiment of Figure 1, the retainer 16 is adapted to allow a user of the endoscope 10 to manually manipulate the handle 18 of the endoscope adjunct device 20 to activate the endoscope adjunct device 20 while the handle 18 is attached to the retainer 16. In one variation, the handle 18 of endoscope adjunct device 20 includes a spool 24 with two ends 26 and a recessed center 28, wherein the recessed center 28 is manually attachable to, and manually detachable from, the retainer 16. The two ends 26 of the spool 24 straddle the retainer 16 to prevent back and forth slippage of the spool 24 in the retainer 16. The user moves the thumb ring 30 of the handle 18 with respect to the spool 24 of the handle 18 to activate the end effector (not shown) at the distal end portion of the cable 22 while the handle 18 is attached to the retainer 16 (or with the handle 18 removed from the retainer 16 and the spool 24 grasped by two fingers of the user). The unnumbered arrow in Figure 1 extending from proximate the retainer 16 towards the recessed center 28 of the spool 24 of the handle 18 of the endoscope adjunct device 20 indicates the direction the endoscope adjunct device 20 is moved to remove it from the retainer 16. Other variations are left to the artisan.

In one extension of the first expression of the embodiment of Figure 1, the endoscope 10 also includes an endoscope flexible tube 32 having a proximal end 34 which is attached to the endoscope handpiece 12 and which is in communication with the working channel opening 14. The endoscope flexible tube 32 also has a distal end portion 36 which is insertable into a body passageway of a patient. In one variation, the endoscope 10 includes knobs 38 allowing the user to manipulate the distal end portion 36 of the endoscope flexible tube 32 and buttons 40 allowing the user to deliver air or water to the distal end portion 36, to supply suction to the distal end portion 36, to take photo snapshots from the distal end portion 36, etc. In one modification, the endoscope 10 includes monitor cabling 42 shown, in Figure 1, extending from the endoscope handpiece 12 to a monitor (not shown). Typically, a "lens cap" (not shown) covers the working channel opening 14 when the opening is not in use. Examples of endoscopes include, without limitation, gastroscopes and colonoscopes.

A second expression of the embodiment of Figure 1 is for an endoscope component 44 including an endoscope-handpiece retainer 16 attachable to an endoscope handpiece 12, wherein the endoscope handpiece 12 includes a working channel opening 14, and wherein the endoscope-handpiece retainer 16 is adapted to manually attach thereto, and manually remove therefrom, a handle 18 of an endoscope adjunct device 20 having a cable 22 insertable into the working channel opening 14.

In one variation of the second expression of the embodiment of Figure 1, the endoscope-handpiece retainer 16 is manually attachable to, and manually removable from, the endoscope handpiece 12. In one modification, a Velcro® strap 46 bonded to the endoscope handpiece 12 engages a Velcro® pad (not shown) bonded to the endoscope-handpiece retainer 16. In another modification, the retainer is attached to a strap having Velcro® -strap free ends which allow strap attachment to, and strap removal from, the endoscope handpiece. It is noted that a Velcro® attachment is a hook and loop attachment. Other modifications are left to the artisan.

In one arrangement of the second expression of the embodiment of Figure 1, the endoscope-handpiece retainer 16 is adapted to allow a user of the endoscope 10, when the endoscope-handpiece retainer 16 is attached to the endoscope handpiece 12, to manually manipulate the handle 18 of the endoscope adjunct device 20 to activate the endoscope adjunct device 20 while the handle 18 is attached to the endoscope-handpiece retainer 16. It is noted that the applications, employments, extensions, etc. of the first expression of the embodiment of Figure 1 are equally applicable to the second expression of the embodiment of Figure 1.

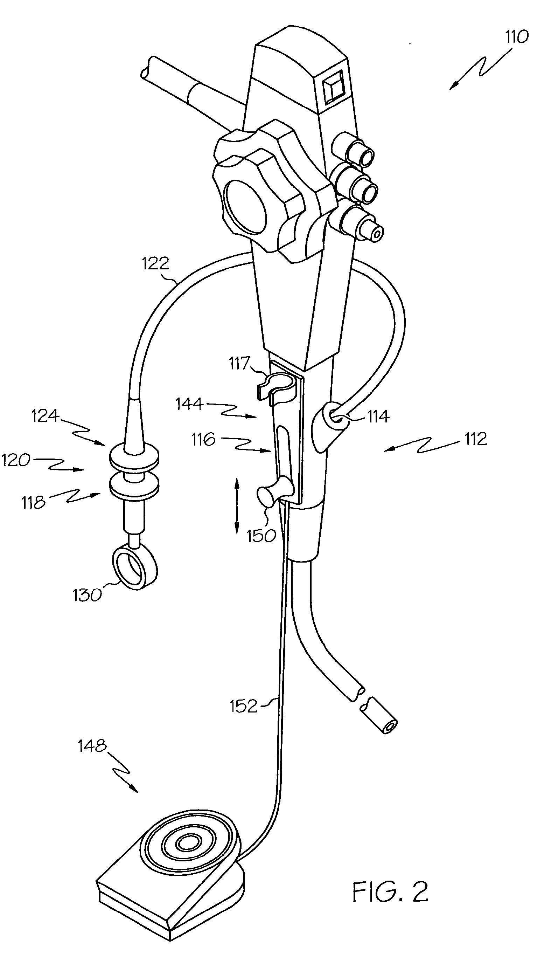

A second embodiment of the invention is shown in Figure 2. A first expression of the embodiment of Figure 2 is for an endoscope 110 including an endoscope handpiece 112 having a working channel opening 114 and an endoscope-adjunct-device actuation carriage 116. The endoscope-adjunct-device actuation carriage 116 is adapted to manually attach thereto, and manually remove therefrom, a handle 118 of an endoscope adjunct device 120 having a cable 122 insertable into the working channel opening 114. The endoscope-adjunct-device actuation carriage 116 is adapted to allow a user of the endoscope 110 to remotely manipulate the handle 118 of the endoscope adjunct device 120 to activate the endoscope adjunct device 120 when the handle 118 is attached to the endoscope-adjunct-device actuation carriage 116.

It is noted that a user does not touch a handle to be said to remotely manipulate the handle. In one example, the endoscope 110 includes a user-depressible pedal/button 148. In this example, the endoscope-adjunct-device actuation carriage 116 includes a movable actuator 150 operatively connectable to the handle 118 and spaced apart from, and operatively connected to, the user-depressible pedal/button 148. The term "pedal/button" includes a foot-depressed, spring-biased floor pedal (shown in figure 2) and a finger or thumb-depressed, spring-biased button on the endoscope handpiece. A control cable 152 operatively connects the pedal/button 148 to the movable actuator 150. The unnumbered double-headed arrow in Figure 2 indicates the back-and-forth direction of movement of the movable actuator 150. The unattached handle 118 shown in Figure 2 would have its spool 124 held by the retainer 117 of the endoscope-adjunct-device actuation carriage 116 and have its thumb ring 130 surround the movable actuator 150 to have the handle 118 be attached to the endoscope-adjunct-device actuation carriage 116. Other examples are left to the artisan.

The employments and extensions of the first expression of the embodiment of Figure 1 are equally applicable to the first expression of the embodiment of Figure 2.

A second expression of the embodiment of Figure 2 is for an endoscope component 144 including an endoscope-adjunct-device actuation carriage 116 attachable to an endoscope handpiece 112. The endoscope handpiece 112 includes a working channel opening 114. The endoscope-adjunct-device actuation carriage 116 is adapted to manually attach thereto, and manually remove therefrom, a handle 118 of an endoscope adjunct device 120 having a cable 122 insertable into the working channel opening 114. The endoscope-adjunct-device actuation carriage 116 is adapted to allow a user of the endoscope 110 to remotely manipulate the handle 118 of the endoscope adjunct device 120 to activate the endoscope adjunct device 120 when the handle 118. is attached to the endoscope-adjunct-device actuation carriage 116.

In one variation of the second expression of the embodiment of Figure 2, the endoscope-adjunct-device actuation carriage 116 is manually attachable to, and manually removable from, the endoscope handpiece 112. In one modification, a Velcro® pad (not shown) is bonded to the endoscope handpiece 112 and engages a Velcro® pad (not shown) bonded to the endoscope-adjunct-device actuation carriage 116. Other modifications are left to the artisan.

The employments and extensions of the first expression of the embodiment of Figure 1 and the examples of the first expression of the embodiment of Figure 2 are equally applicable to the second expression of the embodiment of Figure 2. In one arrangement, the endoscope component 144 also includes the user-depressible pedal/button 148.

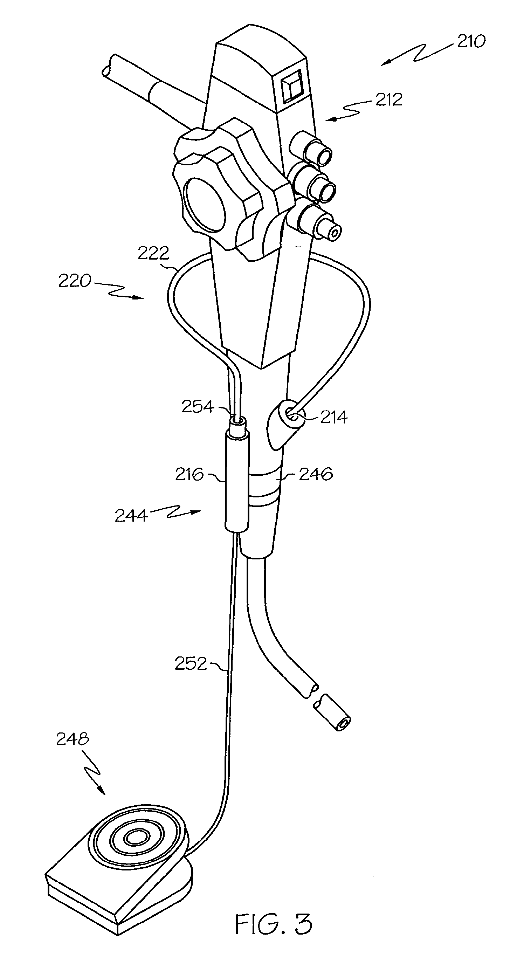

A third embodiment of the invention is shown in Figure 3. A first expression of the embodiment of Figure 3 is for an endoscope 210 including an endoscope handpiece 212 having a working channel opening 214 and an endoscope-adjunct-device actuation cylinder 216. The endoscope-adjunct-device actuation cylinder 216 is adapted to manually operatively attach thereto, and manually operatively remove therefrom, a proximal end 254 of a cable 222 of an endoscope adjunct device 220, wherein the cable 222 has a distal end insertable into the working channel opening 214. The endoscope-adjunct-device actuation cylinder 216 is adapted for remote activation by a user of the endoscope 210.

In one example of the first expression of the embodiment of Figure 3, the endoscope 210 includes a user-depressible pedal/button 248 spaced-apart from, and operatively connected to, the endoscope-adjunct-device actuation cylinder 216. In this example, a control cable 252 operatively connects the pedal/button 248 to the endoscope-adjunct-device actuation cylinder 216. In another example, not shown, the cylinder is a pneumatic cylinder controlled by a pushbutton.

The employments and extensions of the first expression of the embodiment of Figure 1 are equally applicable to the first expression of the embodiment of Figure 3.

A second expression of the embodiment of Figure 3 is for an endoscope component 244 including an endoscope-adjunct-device actuation cylinder 216 attachable to an endoscope handpiece 212. The endoscope handpiece 212 includes a working channel opening 214. The endoscope-adjunct-device actuation cylinder 216 is adapted to manually attach thereto, and manually remove therefrom, a proximal end 254 of a cable 222 of an endoscope adjunct device 220, wherein the cable 222 has a distal end insertable into the working channel opening 214. The endoscope-adjunct-device actuation cylinder 116 is adapted for remote activation by a user of the endoscope 210.

In one variation of the second expression of the embodiment of Figure 3, the endoscope-adjunct-device actuation cylinder 216 is manually attachable to, and manually removable from, the endoscope handpiece 212. In one modification, a strap 246 is attached to the endoscope-adjunct-device actuation cylinder 216 and has Velcro® strap free ends for use in attaching the cylinder 216 to, and removing the cylinder 216 from, the endoscope handpiece 212. Other modifications are left to the artisan.

The employments and extensions of the first expression of the embodiment of Figure 1 and the examples of the first expression of the embodiment of Figure 3 are equally applicable to the second expression of the embodiment of Figure 3. In one arrangement, the endoscope component 244 also includes the user-depressible pedal/button 248.

In any or all of the previously-described expressions of the embodiments of Figures 1-3, an optional scope holder (not shown) can be employed to hold the endoscope handpiece when the distal end of the endoscope flexible tube is at a target site to further facilitate use of the endoscope adjunct device by a single endoscope user.

Several benefits and advantages are obtained from one or more of the expressions of embodiments of the invention. In one application of the first and/or second expression of the first embodiment, by having a retainer which holds the handle of an existing endoscope adjunct device, a single user of the endoscope can operate the endoscope and more conveniently operate the endoscope adjunct device. In one application of the first and/or second expression of the second embodiment, by having an endoscope-adjunct-device actuation carriage, a single user of the endoscope can operate the endoscope and remotely manipulate the handle of the endoscope adjunct device to activate the device. In one application of the first and/or second expression of the third embodiment, by having an endoscope-adjunct-device actuation cylinder, a single user of the endoscope can operate the endoscope and remotely manipulate the cable of a handle-less endoscope adjunct device to activate the device.

While the present invention has been illustrated by a description of several expressions of embodiments and examples, etc. thereof, it is not the intention of the applicants to restrict or limit the spirit and scope of the appended claims to such detail. Numerous other variations, changes, and substitutions will occur to those skilled in the art without departing from the scope of the invention. It will be understood that the foregoing description is provided by way of example, and that other modifications may occur to those skilled in the art without departing from the scope and spirit of the appended Claims.

## Claims

1. An endoscope comprising an endoscope handpiece including a working channel opening and a retainer, wherein the retainer is adapted to manually attach thereto, and manually remove therefrom, a handle of an endoscope adjunct device having a cable insertable into the working channel opening.

2. The endoscope of claim 1, wherein the endoscope adjunct device is chosen from the group consisting of a grasper, biopsy forceps, and a snare.

3. The endoscope of claim 1, wherein the retainer is adapted to allow a user of the endoscope to manually manipulate the handle of the endoscope adjunct device to activate the endoscope adjunct device while the handle is attached to the retainer.

4. The endoscope of claim 1, also including an endoscope flexible tube having a proximal end which is attached to the endoscope handpiece and which is in communication with the working channel opening and having a distal end portion which is insertable into a body passageway of a patient.

5. An endoscope component comprising an endoscope-handpiece retainer attachable to an endoscope handpiece, wherein the endoscope handpiece includes a working channel opening, and wherein the endoscope-handpiece retainer is adapted to manually attach thereto, and manually remove therefrom, a handle of an endoscope adjunct device having a cable insertable into the working channel opening.

6. The endoscope component of claim 5, wherein the endoscope-handpiece retainer is manually attachable to, and manually removable from, the endoscope handpiece.

7. The endoscope component of claim 5, wherein the endoscope adjunct device is chosen from the group consisting of a grasper, biopsy forceps, and a snare.

8. The endoscope component of claim 5, wherein the endoscope-handpiece retainer is adapted to allow a user of the endoscope, when the endoscope-handpiece retainer is attached to the endoscope handpiece, to manually manipulate the handle of the endoscope adjunct device to activate the endoscope adjunct device while the handle is attached to the endoscope-handpiece retainer.

9. An endoscope comprising an endoscope handpiece including a working channel opening and an endoscope-adjunct-device actuation carriage, wherein the endoscope-adjunct-device actuation carriage is adapted to manually attach thereto, and manually remove therefrom, a handle of an endoscope adjunct device having a cable insertable into the working channel opening, and wherein the endoscope-adjunct-device actuation carriage is adapted to allow a user of the endoscope to remotely manipulate the handle of the endoscope adjunct device to activate the endoscope adjunct device when the handle is attached to the endoscope-adjunct-device actuation carriage.

10. The endoscope of claim 9, wherein the endoscope adjunct device is chosen from the group consisting of a grasper, biopsy forceps, and a snare.

11. An endoscope component comprising an endoscope-adjunct-device actuation carriage attachable to an endoscope handpiece, wherein the endoscope handpiece includes a working channel opening, wherein the endoscope-adjunct-device actuation carriage is adapted to manually attach thereto, and manually remove therefrom, a handle of an endoscope adjunct device having a cable insertable into the working channel opening, and wherein the endoscope-adjunct-device actuation carriage is adapted to allow a user of the endoscope to remotely manipulate the handle of the endoscope adjunct device to activate the endoscope adjunct device when the handle is attached to the endoscope-adjunct-device actuation carriage.

12. An endoscope comprising an endoscope handpiece including a working channel opening and an endoscope-adjunct-device actuation cylinder, wherein the endoscope-adjunct-device actuation cylinder is adapted to manually operatively attach thereto, and manually operatively remove therefrom, a proximal end of a cable of an endoscope adjunct device, wherein the cable has a distal end insertable into the working channel opening, and wherein the endoscope-adjunct-device actuation cylinder is adapted for remote activation by a user of the endoscope.

13. An endoscope component comprising an endoscope-adjunct-device actuation cylinder attachable to an endoscope handpiece, wherein the endoscope handpiece includes a working channel opening, wherein the endoscope-adjunct-device actuation cylinder is adapted to manually operatively attach thereto, and manually operatively remove therefrom, a proximal end of a cable of an endoscope adjunct device, wherein the cable has a distal end insertable into the working channel opening, and wherein the endoscope-adjunct-device actuation cylinder is adapted for remote activation by a user of the endoscope.
